# EUROPEAN PATENT APPLICATION

(11) **EP 3 991 720 A1**
(43) Date of publication of application: **04.05.2022**
(21) Application number: 20209795.2
(22) Date of filing: 25.11.2020
(51) Int. Cl.: A61K 9/08, A61K 9/16, A61K 47/26, A61P 1/10, A61K 47/10

(54) **DRY COMPOSITION FOR DISSOLVING IN WATER**

(30) Priority: 30.10.2020 PL 43583120
(71) Applicant: Biogliko Spolka Z Ograniczona Odpowiedzialnoscia, 02-739 Warsaw (PL)
(72) Inventor: Klocek, Jacek, 00-785 Warsaw (PL); Ledwon, Tomasz, 05-100 Nowy Dwor Mazowiecki (PL)
(74) Representative: Kondrat, Mariusz

(57) **Abstract**

The subject of invention is a dry composition for dissolving in water containing an osmotic agent, characterised in that it comprises glucose, one or more sugar alcohols and one or more mucoprotectants, for use as supportive product for the organism of a patient preparing for endoscopy, wherein aqueous solution of the composition is a clear solution of osmolarity of > 170 mOsm/L.

## Description

The subject of the invention is a dry composition for dissolving in water.

Endoscopy (rectoscopy, sigmoidoscopy, colonoscopy) provides the basis for diagnosis of colorectal cancer - it allows for tumour detection, tumour specimen collection and assessment of the remaining parts of the intestine. The procedure of preparation for this examination consists in proper cleansing of the bowel, which is achieved, inter alia, by prolonged (even up to 24 hours) fasting and concurrent intake of strong laxatives. The guidelines of the European Society of Gastrointestinal Endoscopy (ESGE), American Society of Gastrointestinal Endoscopy (ASGE), American Society of Gastroenterology (ACG) and American Gastroenterology Association (AGA) concerning colonoscopy recommend consumption of residue-free or liquid food before the examination as well as the use of adequate products (mainly polyethylene glycol) administered according to a split-dose regimen (a method based on division of the total dose of the product into portions taken on the day before colonoscopy and on the day of its performance). Alternatively, if colonoscopy is performed in the afternoon, the laxative may be taken on the day of examination only.

Regardless of the mechanism of action of known laxatives, these products induce a diarrhoea-like condition, which additionally leads to a loss of minerals and water. Furthermore, people using the above-mentioned products frequently complain of nausea.

Proper preparation for the examination increases significantly the chance of detecting colorectal cancers and polyps, and reduces the discomfort experienced by the examined person. There is a number of factors that increase the risk of suboptimal bowel cleansing. These include metabolic disorders, in particular metabolic disorders that may lead to hypoglycaemic episodes (e.g. diabetes mellitus, hypothyroidism, hypopituitarism and adrenocortical insufficiency, reactive hypoglycaemia).

In patients with metabolic disorders, preparation for the procedure requires a change in their medication regimen and causes a risk of hypoglycaemia (or other blood glucose level disorders) with all consequences of these disorders, from discomfort to - in the most extreme cases - life-threatening conditions. A special group with increased risk of inadequate preparation for the examination are diabetic patients (in particular type 2 diabetes patients). This is due to two types of causes: firstly, one of diabetes complications is microneuropathy that impairs intestinal peristalsis, which leads to retention of the intestinal content; secondly, glucose level drops lead to deviations from the proper conduct of the preparation procedure. In patients in whom endoscopy is carried out under anaesthesia fluctuations of blood glucose levels are an additional risk factor that prevents normal conduct of anaesthesia. Therefore, in patients at particular risk of complications, preparation is not carried out on an outpatient basis and hospitalization may be necessary, which is associated with additional costs for the healthcare system.

Proper cleansing of the bowel is a prerequisite for high quality results. Inadequate preparation leads to prolongation of the time of the procedure and sometimes the entire procedure has to be repeated. This results in a number of negative effects, including the following: higher complication rate, failure to detect lesions in early stages of cancer development and an increase in overall costs for the healthcare system. Professional literature indicates that diabetes is one of the main risk factors for inadequate bowel cleansing for endoscopic examination (Hassan C, Fuccio L, Bruno M, Pagano N, Spada C, Carrara S, Giordanino C, Rodonotti E, Curcio G, Dulbecco P, Fabbri C, Della Casa D, Maiero S, Simone A, Iacopini F, Feliciangeli G, Manes Gm Rinaldi A, Zullo A, Rogai F, Recipi A. A predictive model identifies patients most likely to have inadequate bowel preparation for colonoscopy. Clin Gastroenterol Hepatol. 2012; 10(5): 501-506*;* Chung YW, Han DS., Park KH, Kim KO, Park CH, Hahn T, Yoo KS, Park SH, Kim JH, Park CK. Patient factors predictive of inadequate bowel preparation using polyethylene glycol: a prospective study in Korea. J Clin Gastroenterol. 2009; 43(5): 448-452*;* Taylor C, Schubert ML. Decreased efficacy of polyethylene glycol lavage solution (Golytely) in the preparation of diabetic patients for outpatient colonoscopy: a prospective and blinded study. Am J Gastroentero. 2001; 96(3): 710-714). At present, there is no product specifically intended for use in preparation for endoscopy and addressed to patients with metabolic diseases (especially diabetes), and the methods of hypoglycaemia prevention recommended for these patients are differentiated and described in an ambiguous way. In the absence of a standardized approach to the management of glucose level drops and other problems that may occur during bowel cleansing, patients make many mistakes that result in inadequate bowel cleansing before the procedure.

As regards the agents that facilitate preparation of the patient for endoscopic examination, the state of the art includes numerous bowel cleansing compositions based on one or more strong laxatives. For example, patents EP1567193, EP2014304, PL220812, PL216059 disclose compositions that contain polyethylene glycol (PEG) as the laxative and that effectively cleanse the intestine. Additionally, compositions for colon cleansing often contain electrolytes (EP2683375 B1). Application WO2016208781, on the other hand, discloses a composition for cleansing of the intestinal lumen, containing PEG in combination with bisacodyl as laxatives.

From the state of the art, compositions containing plant extracts with a protective effect on gastrointestinal mucous membranes are known. From documents DE3721038A1 and US4150123A, compositions containing Iceland moss extract are known. However they are intended for use in the treatment of peptic ulcer disease.

From the state of the art, supportive compositions for the human body are known, used in various pathological and physiological conditions, such as amino acid mixtures for athletes. From document US2014255371A, an alkalescent composition is known, intended for use during exercise to improve muscle efficiency, to accelerate muscle regeneration and to improve exertional endurance, containing calcium oxide at a concentration of 500-600 mg/l and a flavouring substance. In a preferred embodiment, the disclosed composition contains plant extracts, including a ginger extract. In addition, in a preferable variant, the alkalescent composition contains a mixture of amino acids, amino acid precursors and amino acid derivatives, in particular 5-HTP, arginine, beta-alanine, carnitine fumarate, citrulline malate, glutamine peptide, glycine, L-alanine, L-arginine, L-arginine hydrochloride, L-histidine, L-methionine, L-lysine hydrochloride, L-phenylalanine, leucine ethyl ester, L-glutamine, L-isoleucine, L-teanine, L-thyrosine, phenylalanine, taurine, trimethylglycine, tryptophan, tyrosine, L-camitine, L-camosine, glutamine alpha-ketoglutarate and alpha-L-polylactate. The composition contains preferably one or more sweeteners, which may include potassium acesulfame, aspartame, cane sugar, corn syrup, crystalline fructose, dextrose, D-ribose, fructose, glucose, glucose-fructose syrup, high fructose corn syrup, high fructose content liquid, sugar, honey, maltodextrin, sorbitol, stevia, sucralose, sucrose, trehalose, truvia or xylitol.

Additionally, amino acid compositions are known from the state of the art that support the treatment of diabetes. In this context, document US2015174088 AA discloses a dietary supplement for supportive treatment of diabetes, containing one or more alpha-ketoacids and / or their salts selected from the group including alpha-ketoglutarate, alpha-ketoisocaproate, alpha-ketoisovalerate and alpha-keto-beta-methylvalerate and /or their salts. Essentially, this product does not contain nitrogen. In a preferred embodiment, the above-mentioned food supplement contains further additives selected from the groups of carbohydrates, fats and oils, vitamins, antioxidants, minerals and trace elements, preservatives, food colourants, sweeteners, flavour enhancers and flavours.

However, amino acid compositions are not used in patients' preparation for endoscopy.

Known compositions are unable to meet comprehensive needs of a patient preparing for gastrointestinal medical procedures (e.g. endoscopy or colonoscopy).

The aim of the invention was to provide a new composition supporting the patient's organism preparing for medical procedures in the areas of gastroenterology and endoscopy that would reduce the risk of hypoglycaemia.

The subject of the invention is a dry composition for dissolving in water containing an osmotic agent, characterized in that it comprises glucose, one or more sugar alcohols and one or more mucoprotectants, for use as supportive product for the patient's organism preparing for endoscopy, wherein aqueous solution of the composition is a clear solution of osmolarity of > 170 mOsm/L.

In a preferred embodiment, the composition comprises the following components per litre of aqueous solution of the composition:
a) one or more sugar alcohols at an amount of 1 g to 100 g
b) glucose at an amount of 10 g to 100 g
c) one or more mucoprotectants at an amount of 10 mg to 5 g

In a preferred embodiment, one or more sugar alcohols are selected from the group comprising sorbitol, xylitol or a combination thereof.

In a preferred embodiment, one or more mucoprotectants are selected from the group comprising hyaluronic acid, Iceland moss extract or a combination thereof.

In a preferred embodiment, the composition comprises one or more amino acids.

In a preferred embodiment, one or more amino-acids are selected from the group comprising L-arginine alpha-ketoglutarate, beta-alanine, arginine, glutamine, tryptophan or a combination thereof.

In a preferred embodiment, the composition comprises one or more antiemetics and anti-nausea substances selected from the group comprising ginger rhizome extract, licorice root extract, chamomile extract, peppermint oil, vitamin B6.

In a preferred embodiment, composition comprises one or more substances selected from a group including flavouring substances, buffering agents or combinations thereof.

The patient is preferably a patient with metabolic disorders.

A metabolic disorder is preferably selected from the group comprising diabetes mellitus, hypothyroidism, hypopituitarism, adrenocortical insufficiency, reactive hypoglycaemia.

In a preferred embodiment, after dissolving the composition in water, aqueous solution of the composition, according to the invention, has osmolarity of >200 mOsm/L.

In a preferred embodiment, after dissolving the composition in water, aqueous solution of the composition, according to the invention, has osmolarity of >700 mOsm/L.

In a preferred embodiment, pH of the aqueous solution of the composition is <7.

The composition is preferably dissolved in 50 to 500 ml of water.

In a preferred embodiment, the composition is dissolved in 150 ml of water.

This invention provides the following advantages:
- reduces the risk of hypoglycaemia in patients with metabolic diseases (e.g. type 2 diabetes), which may be the result of dietary restrictions within 24 hours prior to the procedure;
- provides standardized and predictable elevation of blood glucose level without reducing the level of bowel cleanliness;
- facilitates patient's preparation for procedures by reducing anxiety and stress associated with these procedures and by reducing muscle tension, vomiting reflexes as well as gastrointestinal tonisation;
- the ingredients of the composition do not interfere with drugs commonly used in metabolic diseases;
- has a comprehensive effect by supplementing carbohydrates and providing an environment that maximizes their absorption in the gastrointestinal tract;
- contains plant extracts to prevent nausea and to reduce the production of bile acids that adversely affect the level of bowel cleanliness during the procedure.

The invention is presented in embodiments.

### Embodiment 1

The composition according to the invention is used to support patient's organism during preparation for endoscopic examinations, surgery procedures, etc. - if the patient is using osmotic products and/or has to refrain from food intake for a longer period.

The composition according to the invention is intended for each patient to reduce his/her discomfort associated with preparing for examinations. However, its use is particularly beneficial for people who are sensitive to sugar level drops due to metabolic disorders (e.g. diabetes, hypothyroidism, hypopituitarism, adrenocortical insufficiency or reactive hypoglycaemia), hormonal disorders or other causes.

The composition according to the invention has a comprehensive action by carbohydrate supplementation, providing an environment that maximizes their absorption in the gastrointestinal tract, and at the same time providing correction of the level of electrolytes that are being lost during the preparation for the examination.

Because of its osmolarity, the composition according to the invention enables patient's bowel cleansing, thus facilitating its preparation for planned endoscopy procedures (e.g. colonoscopy, rectoscopy, sigmoidoscopy). Additionally, the composition helps to prepare the patient for the procedures by reducing anxiety and stress associated with these procedures and by reducing muscle tension, vomiting reflexes as well as gastrointestinal tonisation.

The composition according to the invention contains naturally-occurring substances of high bioavailability and readily soluble in water, whose concentrations do not exceed the maximum doses (i.e. maximum safe doses) and which do not interfere with the medicines commonly used in metabolic diseases.

The composition according to the invention has a loose form (e.g. powder, granulate). It is packed into single-dose sachets and can be dissolved in water, e.g. in a glass of water.

In its basic variant, the dry composition to be mixed with water according to the invention contains glucose, one sugar alcohol affecting osmolarity to help bowel cleansing and one or more mucoprotectants. In this preferred and non-limiting embodiment, the composition according to the invention comprises:
15000 mg (i.e. 15 g) of glucose,
5000 mg (i.e. 5 g) of sorbitol,
and 50 mg of hyaluronic acid,

In this preferred embodiment, pH of the composition is 5.4.

Wherein this composition according to the invention has the form of a powder for preparation of an aqueous solution, and this powder is contained in a container of any form (e.g. a bottle, a sachet, etc.).

Wherein in this embodiment the above-mentioned composition is dissolved in 150 ml of water at room temperature (i.e. 15-25°C) before consumption by the patient. However, the water for dissolution of the composition according to the invention does not need to be warm, as the analyses carried out have shown that all ingredients of the composition dissolve already at 10°C.

### Embodiment 2

Powder composition as in embodiment 1, except that it comprises:
15000 mg (i.e. 15 g) of glucose,
5000 mg (i.e. 5 g) of xylitol,
and 53.3 mg of Iceland moss extract, *(Cetraria islandica),*

Form: powder for preparation of aqueous solution. The composition is intended to be dissolved in 150 ml of water. In this preferred embodiment, pH of the composition is 5.8.

### Embodiment 3

Composition in the form of powder for preparation of aqueous solution as in embodiment 1, except that one sachet with powder for solution in 150 ml of water contains:
1500 mg (i.e. 10 g/L) of glucose,
15000 mg (i.e. 100 g/L) of sorbitol,
750 mg (i.e. 5g/L) of Iceland moss extract, *(Cetraria islandica),*

In this preferable embodiment, pH of the composition is 5.9.

### Embodiment 4

Composition in the form of powder for preparation of aqueous solution as in embodiment 1, except that one sachet with powder for solution in 150 ml of water contains:
7500 mg (i.e. 50 g/L) of glucose,
150 mg (i.e. 1 g/L) of xylitol,
1.5 mg (i.e. 10 mg/L) of hyaluronic acid,

In this preferred embodiment, pH of the composition is 5.1.

### Embodiment 5

Composition according to the invention in the form of powder for preparation of aqueous solution, where the powder is contained in one sachet and is intended to be dissolved in 150 ml of water, with one sachet containing:
15000 mg (i.e. 100 g/L) of glucose,
1500 mg (i.e. 10 g/L) of xylitol,
7500 mg (i.e. 50 g/L) of sorbitol,
150 mg (i.e. 1g/L) of Iceland moss extract *(Cetraria islandica),*
300 mg (i.e. 2 g/L) of hyaluronic acid,
120 mg of licorice root extract.

In this preferred embodiment, pH of the composition is: 5.7.

### Embodiment 6

Composition according to the invention in the form of powder for preparation of aqueous solution, where the powder is contained in one sachet and is intended to be dissolved in 150 ml of water, with one sachet containing:
15000 mg of glucose,
5000 mg of sorbitol,
125 mg of ginger extract, DER 2-4:1,
53.22 mg of Iceland moss extract,
50 mg of hyaluronic acid,
400 mg of glutamine,
400 mg of arginine,
1mg of vitamin B6.

Wherein ginger rhizoma (*Zingiberis rhizoma*) extract has a DER of 2-4:1. In contrast, the Iceland moss *(Cetraria islandica*) extract is a concentrated aqueous extract.

### Embodiment 7.

Composition according to the invention in the form of powder for preparation of aqueous solution, where the powder is contained in one sachet and is intended to be dissolved in 150 ml of water, with one sachet containing:
7500 mg of glucose,
1000 mg of sorbitol,
60 mg of chamomile extract,
65 of peppermint oil,
53.33 mg of Iceland moss extract,
50 mg of hyaluronic acid,
400 mg glutamine,
400 mg of arginine.

### Embodiment 8.

Composition according to the invention in the form of powder for preparation of aqueous solution, where the powder is contained in one sachet and is intended to be dissolved in 150 ml of water, wherein one sachet contains:
15000 mg of glucose,
5000 mg of sorbitol,
125 mg of ginger extract,
53.33 mg of Iceland moss extract,
166.67 mg of L-tryptophan,
50 mg of hyaluronic acid,
800 mg of beta-alanine,
1000 mg of L-arginine alpha-ketoglutarate.

In this preferred embodiment, pH is 5.4.

### Embodiment 9.

Composition according to the invention in the form of powder for preparation of aqueous solution, where the powder is divided into two portions, each of them in a separate sachet.

Sachet A contains:
125 mg of ginger extract, DER 2-4:1,
53.33 mg of Iceland moss extract,
166.67 mg of L-tryptophan,
800 mg of beta-alanine,
1000 mg of L-arginine alpha-ketoglutarate,
pH of the composition in sachet A is 5.6.

Sachet B contains:
15000 mg of glucose,
5000 mg of sorbitol.
pH of the composition in sachet B is 6.7.

Each of these compositions is a powder for solution in 150 ml of water.

### Embodiment 10.

Analysis of the osmolarity of the composition according to the invention. Two sample compositions according to the invention were selected for analysis, hereinafter referred to as composition 1 and composition 2, whose composition is presented in Tables 1 and 2.

**Table 1: Quantitative and qualitative composition of composition 1**

| Composition 1 | | |
|---|---|---|
| **INGREDIENTS** | **Daily portion (mg) - 3 sachets** | **Portion persachet (mg)** |
| ginger extract, DER 2-4:1 | 375 | 125.00 |
| Iceland moss extract | 160 | 53.33 |
| L-tryptophan | 500 | 166.67 |
| Hyaluronic acid | 150 | 50.00 |
| Beta-alanine | 2400 | 800.00 |
| Alpha-arginine L-ketoglutarate | 3000 | 1000.00 |
| glucose | 45000 | 15000.00 |
| sorbitol | 15000 | 5000.00 |
| total | | 22195 |

pH of the composition 1 is 5.4

**Table 2: Quantitative and qualitative composition of composition 2**

| Composition 2 | | |
|---|---|---|
| **INGREDIENTS** | **Daily portion (mg) - 3 sachets** | **Portion per sachet (mg)** |
| ginger extract, DER 2-4:1 | 375 | 125.00 |
| Iceland moss extract | 160 | 53.33 |
| L-tryptophan | 500 | 166.67 |
| Beta-alanine | 2400 | 800.00 |
| Alpha-arginine L-ketoglutarate | 3000 | 1000.00 |
| total | 6435 | 2145 |

pH of the composition 2 is 5.6

Composition 1 is a complete composition as shown in embodiment 8, whereas composition 2 corresponds to the content of sachet A in the variant of composition divided into two sachets presented in embodiment 9.

A flavouring additive based on concentrated fruit juice with a low content of sugar naturally occurring therein was added to each composition.

In this embodiment, 3 different flavours are used:
1. Citrus flavour
2. Apple flavour
3. Multi-fruit flavour

After each flavour variant was added to either composition 1 or composition 2, the following variants of composition were obtained:
- Composition 1, flavour 1, labelled as 1.1.
- Composition 1, flavour 2, labelled as 1.2.
- Composition 1, flavour 3, labelled as 1.3.
- Composition 2, flavour 1, labelled as 2.1.
- Composition 2, flavour 2, labelled as 2.2.
- Composition 2, flavour 3, labelled as 2.3.

After flavour addition, pH of compositions 1.2 and 2.1 was re-tested by random sampling, and pH of composition 1.2 was 4.54 and pH of composition 2.1 was 4.71

Each of these compositions was tested for osmolarity. The test was carried out in an external laboratory with an accredited method. The studies were carried out by Eurofins Bel/Novamann (Bratislava, Slovakia), accreditation number S-106. The density of the samples was calculated with the picometric method (ŠPP 012-F Ph. Eur. method) and osmolarity - with the osmometric method (ŠPP 033-F Ph. Eur. method). The results are presented in Table 3:

**Table 3. Analysis of the osmolarity of the composition according to the invention**

| The variant of the tested composition | Osmolarity (mOsm/L) |
|---|---|
| 1.1. | 781.4 |
| 1.2. | 797.1 |
| 1.3. | 928.2 |
| 2.1. | 201.8 |
| 2.2. | 209.7 |
| 2.3. | 179.5 |

The above analysis shows that the complete composition according to the invention is characterized in that it has a high osmolarity, i.e. osmolarity above 700 mOsm/L, which means that the mucoprotectant and favourable optional components ensure osmolarity within the range of 179-210 mOsm/L.

Accordingly, the composition according to the invention (even in its basic variant) meets the requirement of osmolarity higher than 170 mOsm/L.

In this embodiment, a flavouring additive excipient was used, however other known excipients such as taste masking agents, colourants or buffering agents may also be used in the composition.

### Embodiment 11.

Exemplary product dosing variants are presented in this embodiment.

### Dosage of the product - option 1, i.e. short dosing, during the last 24 hours prior to the planned examination

During the last 24 hours before the planned examination, the patient receives the composition according to the invention, containing glucose, sugar alcohol, amino acids, gastrointestinal mucoprotectants, as well as antiemetics and anti-nausea medicines. The composition according to embodiment 8 was used in this embodiment. A total of 3 sachets is an intended daily dose to be consumed during the last 24 hours before the examination at time intervals and in case when the planned examination is performed under anaesthesia, the patient is instructed to receive the last portion not later than four hours before the planned examination,.

The composition should be taken in the form of solution prepared from the powder contained in the sachet, dissolved in a specified amount of water. The optimum amount of water is 150 ml, however it is recommended to use not less than 100 ml of water. After the composition according to the invention is dissolved in water, a clear solution is obtained.

The product should be dissolved in water at room temperature and may be cooled before drinking.

In this variant, packaging containing composition according to the invention will be offered as a pack of 3 sachets in one package that will also contain instructions for use.

### Dosage of the product - option 2, i.e. long dosing, during 7 days before the examination

In this dosing variation the 2-sachet composition according to embodiment 9 is used, where sachet A contains all ingredients except for glucose and sugar alcohol, and sachet B contains sugar alcohol and glucose.

Wherein, during the last 24 hours, three portions of the composition according to the invention are taken by dissolving sachets A and B in 150 ml of water, where the contents of both sachets can be dissolved together or separately. Wherein it is recommended to dissolve the contents of sachets A and B together, in 150 ml of water.

In contrast, only sachet A is taken for six days preceding the last 24 hours before the examination. The content of the sachet is dissolved in the prespecified amount of water, e.g. 150 ml (although 50 to 500 ml may be used). The prepared solution should be taken in the evening. In this dosing embodiment, one sachet A per day is consumed (the recommended variant), however dosing regimens may be used where up to three sachets A per day are administered.

The product should be dissolved in water at room temperature and may be cooled before drinking.

The composition will be offered to patients as a 12-sachet package (including 9 sachets A and 3 sachets B) in one package, containing also instructions for use. The colour of sachets B may be different from that of sachets A to facilitate differentiation of their content.

### Embodiment 12.

Confirmation of hypoglycaemia prevention by the composition according to the invention.

The composition according to the invention reduces the risk of hypoglycaemia, in particular in patients with metabolic diseases (e.g. type 2 diabetes), which may be the result of dietary restrictions during preparation to endoscopy.

The composition was tested in a group of 53 volunteers (n = 53) with metabolic disorders aged 21-65 years. The volunteers were divided into a study group (n =26) and a control group (n=27). The study group received the composition according to the invention (variant according to embodiment 9 with citrus flavour added), according to the following regimen: sachet A for 6 days and subsequently, sachets A + B administered as recommended during 24 hours preceding the examination. In the second group (control group), a mixture that was neutral for sugar metabolism (placebo) was used, according to the same dosing regimen. A survey was carried out to assess the incidence of subjective sensation of "sugar level drop", the severity of symptoms related to decreased blood glucose levels, the impression of comfort and safety during preparation for endoscopy and the ease of composition administration according to the recommended regimen. The level of stress during the preparation period was also assessed using VAS (visual analogue scale).

In the study group receiving the tested composition according to the invention the following was shown: a better tolerance of blood sugar level fluctuations during the last 24 hours before the examination, a significant decrease of the severity of symptoms related to the episodes of blood glucose level decrease, a higher sense of safety and comfort than in the control group, and significantly reduced anxiety and stress associated with the period of preparation for endoscopy. The ease and comfort of administration of the proposed composition was assessed as very good and its taste as extremely pleasant.

## Claims

1. A dry composition for dissolving in water containing an osmotic agent, **characterised in that** it comprises glucose, one or more sugar alcohols and one or more mucoprotectants, for use as supportive product for the organism of a patient preparing for endoscopy, wherein aqueous solution of the composition is a clear solution of osmolarity of > 170 mOsm/L.

2. The composition according to claim 1, **characterised in that** it comprises the following components per litre of aqueous solution of the composition:
a) one or more sugar alcohols at an amount of 1 g to 100 g
b) glucose at an amount of 10 g to 100 g
c) one or more mucoprotectants at an amount of 10 mg to 5 g

3. Composition according to claim 1 or 2, **characterised in that** one or more sugar alcohols are selected from the group comprising sorbitol, xylitol or a combination thereof.

4. Composition according to any of previous claims 1 to 3, **characterised in that** one or more mucoprotectant agents are selected from a group comprising hyaluronic acid, Iceland moss extract or a combination thereof.

5. Composition according to any of the previous claims 1 to 4, **characterised in that** it comprises one or more amino acids.

6. Composition according to any of the previous claims 1 to 5, **characterised in that** one or more amino-acids are selected from the group comprising L-arginine alpha-ketoglutarate, beta-alanine, arginine, glutamine, tryptophan or a combination thereof.

7. Composition according to any of the previous claims 1 to 6 **characterised in that** it comprises at least one antiemetic and anti-nausea substance selected from the group comprising the ginger rhizome extract, licorice root extract, chamomile extract, peppermint oil, vitamin B6.

8. Composition according to any of the previous claims 1 to 7 **characterised in that** it comprises one or more substances selected from the group including flavouring substances, buffering agents or combinations thereof.

9. Composition according to any of the previous claims 1 to 8 **characterised in that** the patient is a patient with metabolic disorders.

10. Composition according to claim 9 **characterised in that** a metabolic disorder is selected from the group comprising diabetes mellitus, hypothyroidism, hypopituitarism, adrenocortical insufficiency, reactive hypoglycaemia.

11. Composition according to any of the previous claims 1 to 10 **characterised in that** after dissolution in water the aqueous solution of the composition has an osmolarity of >200 mOsm/L.

12. Composition according to claim 11, **characterised in that** after being dissolved in water the aqueous solution of the composition has an osmolarity of >700 mOsm/L.

13. Composition according to any of the previous claims 1 to 12 **characterised in that** pH of the aqueous solution of the composition is <7.

14. Composition according to any of the previous claims 1 to 13 **characterised in that** it is dissolved in 50 to 500 ml of water.

15. Composition according to claim 14 **characterised in that** it is dissolved in 150 ml of water.
